(19) Europäisches Patentamt
European Patent Office
Office européen des brevets

(11) Veröffentlichungsnummer: **0 142 654**
**A1**

(12) # EUROPÄISCHE PATENTANMELDUNG

(21) Anmeldenummer: 84110734.5

(22) Anmeldetag: 08.09.84

(51) Int. Cl.[4]: C 07 D 401/12
A 01 N 43/64

(30) Priorität: 23.09.83 DE 3334409

(43) Veröffentlichungstag der Anmeldung: 29.05.85 Patentblatt 85/22

(84) Benannte Vertragsstaaten: AT BE CH DE FR GB IT LI NL SE

(71) Anmelder: BAYER AG
Konzernverwaltung RP Patentabteilung
D-5090 Leverkusen 1 Bayerwerk(DE)

(72) Erfinder: Kraatz, Udo, Dr.
Andreasstrasse 22a
D-5090 Leverkusen 1(DE)

(72) Erfinder: Büchel, Karl Heinz, Prof.Dr.
Dabringhausener Strasse 42
D-5093 Burscheid(DE)

(72) Erfinder: Hänssler, Gerd, Dr.
Heymannstrasse 40
D-5090 Leverkusen(DE)

(72) Erfinder: Brandes, Wilhelm, Dr.
Eichendorffstrasse 3
D-5653 Leichlingen 1(DE)

(72) Erfinder: Reinecke, Paul, Dr.
Lessingstrasse 11
D-5090 Leverkusen 3(DE)

(54) **Triazolylmethyl-pyridyloxymethyl-carbinol-Derivate.**

(57) Die voliegende Erfindung betrifft neue Triazolylmethyl-pyridyloxymethyl- carbinol-Derivate, ein Verfahren zu ihrer Herstellung sowie ihre Verwendung als Fungizide.

Die Verbindungen der allgemeinen Formel

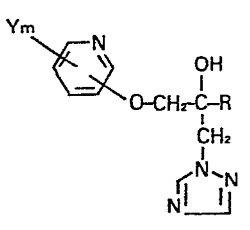

(I)

in welcher R, Y und m die in der Beschreibung angegebene Bedeutung besitzen,

werden durch Umsetzung des entsprechenden Oxirans mit 1,2,4-Triazol in Gegenwart eines Verdünnungsmittels und gegebenenfalls in Gegenwart einer Base erhalten.

Die Wirkstoffe sind für den Gebruach als Pflanzenschutzmittel geeignet und können insbesondere zur Bekämpfung von Pflanzenkrankheiten in Reis-, Getriede-, Gemüse- und Obstanbau Verwendung finden.

EP 0 142 654 A1

Croydon Printing Company Ltd.

BAYER AKTIENGESELLSCHAFT 5090 Leverkusen, Bayerwerk

Konzernverwaltung RP
Patentabteilung

Slr/Ke

Ia

# Triazolylmethyl-pyridyloxymethyl-carbinol-Derivate

Die vorliegende Erfindung betrifft neue Triazolylmethyl-pyridyloxymethyl-carbinol-Derivate, ein Verfahren zu ihrer Herstellung sowie ihre Verwendung als Fungizide.

Es ist bereits bekannt geworden, daß bestimmte, gegebenenfalls im Phenylteil substituierte 3,3-Dimethyl-1-phenoxy-2-(1,2,4-triazol-1-yl-methyl)-2-butanole bzw. 1-Phenoxy-2-phenyl-3-(1,2,4-triazol-1-yl)-2-propanole gute fungizide Eigenschaften aufweisen (vgl. DE-OS 30 18 866 [LeA 22 330]). Die Wirkung dieser Verbindungen ist jedoch, insbesondere bei niedrigen Aufwandmengen und -konzentrationen, nicht immer voll befriedigend.

Es wurden neue Triazolylmethyl-pyridyloxymethyl-carbinol-Derivate der allgemeinen Formel (I),

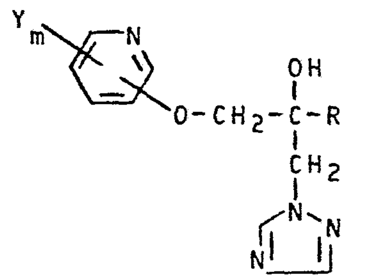

(I)

in welcher

R für gegebenenfalls substituiertes Alkyl, gegebenenfalls substituiertes Cycloalkyl oder gegebenenfalls substituiertes Phenyl steht;

Y für Halogen, Alkyl, Alkoxy oder Cyano steht und

m für die Zahlen 0, 1, 2, 3 oder 4 steht,

und deren Säureadditions-Salze und Metallsalz-Komplexe gefunden.

Die Verbindungen der Formel (I) besitzen ein asymmetrisches Kohlenstoffatom und können deshalb in den beiden optischen Isomerenformen anfallen.

Weiterhin wurde gefunden, daß man die Triazolylmethyl-pyridyloxymethyl-carbinol-Derivate der Formel (I) erhält, wenn man Oxirane der Formel (II),

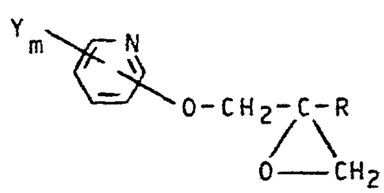

(II)

in welcher

R, Y und m die oben angegebene Bedeutung haben,

mit 1,2,4-Triazol der Formel (III),

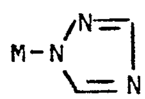

(III)

in welcher

M für Wasserstoff oder ein Alkalimetall steht,

in Gegenwart eines Verdünnungsmittels und gegebenenfalls in Gegenwart einer Base umsetzt und gegebenenfalls anschließend eine Säure oder ein Metallsalz addiert.

Außerdem wurde gefunden, daß die neuen Triazolylmethyl-pyridyloxymethyl-carbinol-Derivate der Formel (I) starke fungizide Eigenschaften aufweisen.

Ueberraschenderweise zeigen die erfindungsgemäßen Triazolylmethyl-pyridyloxymethyl-carbinol-Derivate der Formel (I) bessere fungizide Wirkungen als die oben genannten, aus dem Stand der Technik bekannten 3,3-Dimethyl-1-phenoxy-2-(1,2,4-triazol-1-yl-methyl)-2-butanole bzw. 1-Phenoxy-3-phenyl-3-(1,2,4-triazol-1-yl)-2-propanole, welche konstitutionell und wirkungsmäßig naheliegende Verbindungen sind. Die erfindungsgemäßen Wirkstoffe stellen somit eine Bereicherung der Technik dar.

Die erfindungsgemäßen Triazolylmethyl-pyridyloxymethyl-carbinol-Derivate sind durch die Formel (I) allgemein definiert. In dieser Formel stehen vorzugsweise

R für geradkettiges oder verweigtes Alkyl mit 1 bis 4 Kohlenstoffatomen, für gegebenenfalls einfach oder mehrfach, gleich oder verschieden substituiertes Cycloalkyl mit 3 bis 7 Kohlenstoffatomem, wobei als Substituenten genannt seien : Halogen, Alkyl mit 1 bis 4 Kohlenstoffatomen und Alkoxy mit 1 bis 2 Kohlenstoffatomen, ferner für gegebenenfalls einfach oder mehrfach, gleich oder verschieden substituiertes Phenyl, wobei als Substituenten die unten bei $R^3$ genannten Phenylsubstituenten infrage kommen, sowie für die Gruppierungen

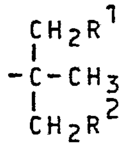 und 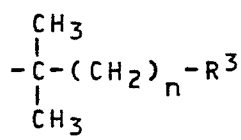 , worin

$R^1$ für Wasserstoff oder Halogen steht;

$R^2$ für Halogen steht und

$R^3$ für Alkyl, Alkoxy und Alkylthio mit jeweils 1 bis 4 Kohlenstoffatomen steht, für Halogenalkoxy und Halogenalkylthio mit jeweils 1 bis 2 Kohlenstoffatomen und 1 bis 5 gleichen oder verschiedenen Halogenatomen, wie Fluor- und Chloratomen, ferner für Alkenyl mit 2 bis 6 Kohlenstoffatomen, für Alkoxycarbonyl mit 1 bis 4 Kohlenstoffatomen im Alkylteil, für Cyano sowie für gegebenenfalls einfach oder mehrfach substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten vorzugsweise ge-

nannt seien : Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, wie insbesondere Fluor- und Chloratomen, Cyclohexyl, Dialkylamino mit 1 bis 4 Kohlenstoffatomen in jedem Alkylteil, Nitro, Cyano, Alkoxycarbonyl mit 1 bis 4 Kohlenstoffatomen im Alkylteil, sowie gegebenenfalls durch Halogen substituiertes Phenyl, und

n für die Zahlen 0, 1 oder 2 steht;

Y für Halogen, geradkettiges oder verzweigtes Alkyl und/oder Alkoxy mit jeweils 1 bis 4 Kohlenstoffatomen sowie Cyano und

m für die Zahlen 0, 1, 2, 3 oder 4.

Besonders bevorzugt sind diejenigen Verbindungen der Formel (I), in denen

R für tert.-Butyl oder Isopropyl steht, für jeweils gegebenenfalls einfach bis dreifach, gleich oder verschieden substituiertes Cyclopropyl, Cyclopentyl oder Cyclohexyl steht, wobei als jeweilige Substituenten genannt seien: Methyl, Ethyl, Isopropyl, Methoxy und Ethoxy, ferner für gegebenenfalls einfach bis dreifach, gleich oder verschieden substituiertes Phenyl steht, wobei als Substituenten genannt seien : Fluor, Chlor, Methyl, Trifluormethyl, Phenyl und Chlorphenyl, sowie für die Gruppierungen

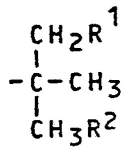 und 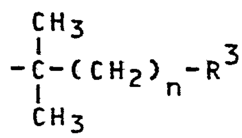 steht, wobei

$R^1$ für Wasserstoff, Fluor oder Chlor steht;

$R^2$ für Fluor oder Chlor steht;

$R^3$ für Methyl, Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano sowie für jeweils gegebenenfalls substituiertes Phenyl, Phenoxy, Phenylthio, Phenylmethoxy oder Phenylmethylthio steht,wobei jeweils als Phenylsubstituenten genannt seien : Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl und

n für die Zahlen 0, 1 oder 2 steht;

Y für Fluor, Chlor, Brom, Iod, Methyl, Ethyl, Methoxy oder Cyano steht und

m für die Zahlen 0, 1, 2, 3 oder 4 steht.

Ganz besonders bevorzugt sind diejenigen Verbindungen der Formel (I), in denen

R für tert.-Butyl oder für gegebenenfalls einfach bis dreifach, gleich oder verschieden substituiertes Phenyl steht, wobei als Substituenten genannt seien : Fluor, Chlor, Methyl, Trifluormethyl, Phenyl und Chlorphenyl, sowie auch für die Gruppierungen

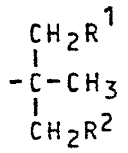 und 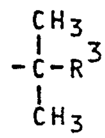 steht, wobei

$R^1$ für Wasserstoff, Fluor oder Chlor steht,

$R^2$ für Fluor oder Chlor steht und

$R^3$ für jeweils gegebenenfalls einfach bis zweifach, gleich oder verschieden substituiertes Phenoxy, Phenylthio, Phenylmethoxy oder Phenylmethylthio steht, wobei jeweils als Phenylsubstituenten genannt seien : Fluor, Chlor, Methyl, Methoxy, Methylthio, Trifluormethyl, Trifluormethoxy, Trifluormethylthio, Methoxycarbonyl und Ethoxycarbonyl;

Y für Fluor, Chlor, Brom oder Iod steht und

m für die Zahlen 0, 1, 2 oder 3 steht.

Bevorzugte erfindungsgemäße Verbindungen sind auch Additionsprodukte aus Säuren und denjenigen Triazolylmethyl-pyridyloxymethyl-carbinol-Derivaten der Formel (I), in denen die Substituenten R und $Y_m$ die Bedeutungen haben, die bereits vorzugsweise für diese Substituenten genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure, sowie Sulfonsäuren, wie z.B. p-Toluolsulfonsäure und 1,5-Naphthalindisulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe und denjenigen Triazolylmethyl-pyridyloxymethyl-carbinol-Derivaten der Formel (I), in denen die Substituenten R und $Y_m$ die Bedeutungen haben, die bereits vorzugsweise für diese Substituenten genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Verwendet man beispielsweise 2-[2-(4-Chlorbenzyloxy)-prop-2-yl]-2-(6-chlorpyridin-2-yloxymethyl)-oxiran und 1,2,4-Triazol als Ausgangsstoffe, so kann der Ablauf des erfindungsgemäßen Verfahrens durch das folgende Formelschema wiedergegeben werden:

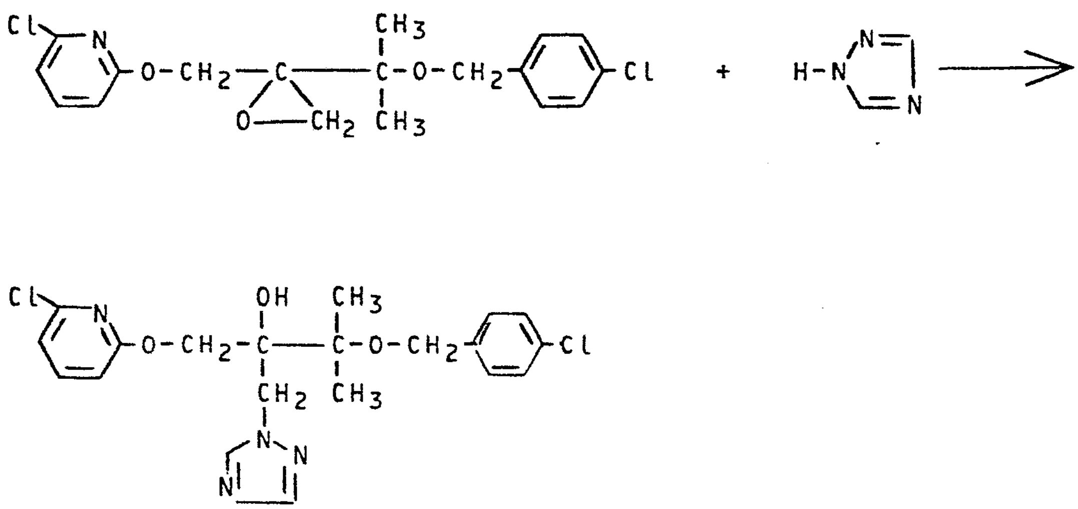

Die bei der Durchführung des erfindungsgemäßen Verfahrens als Ausgangsstoffe zu verwendenden Oxirane sind durch die Formel (II) allgemein definiert. In dieser Formel haben R, Y und der Index m vorzugsweise die Bedeutungen, die bereits im Zusammenhang mit der Beschreibung der erfindungsgemäßen Stoffe der Formel (I) vorzugsweise für diese Substituenten bzw. für den Index m genannt wurden.

Die Oxirane der Formel (II) sind noch nicht bekannt. Sie stellen interessante Zwischenprodukte dar und können in allgemein bekannter Art und Weise erhalten werden, indem man Ketone der Formel (IV),

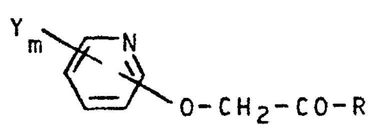

(IV)

in welcher

R, Y und m die oben angegebene Bedeutung haben,

entweder

α) mit Dimethyloxosulfonium-methylid der Formel (V)

$$\overset{\delta+}{(CH_3)_2SO}\overset{\delta-}{CH_2} \qquad (V)$$

in Gegenwart eines Verdünnungsmittels umsetzt,

oder

β) mit Trimethylsulfonium-methylsulfat der Formel (VI)

$$\left[(CH_3)_3S^{(+)}\right] \quad (CH_3SO_4)^{(-)} \qquad (VI)$$

in Gegenwart eines inerten organischen Lösungsmittels sowie in Gegenwart einer Base umsetzt.

Die bei der Herstellung der Oxirane der Formel (II) als Ausgangsstoffe benötigten Ketone der Formel (IV) lassen sich nach im Prinzip bekannten Verfahren herstellen (vgl. z.B. die Herstellungsbeispiele).

Das bei der Verfahrensvariante (α) benötigte Dimethyloxosulfoniummethylid der Formel (V) ist bekannt (vgl. J.Amer. Chem.Soc. 87, 1363-1364 (1965)). Es wird bei der obigen Umsetzung in frisch zubereitetem Zustand verarbeitet, indem man es in situ durch Umsetzung von Trimethyloxosulfoniumiodid mit Natriumhydrid, Natriumamid bzw. Kalium-tert.-butylat in Gegenwart eines Verdünnungsmittels erzeugt.

Das bei der Verfahrensvariante (β) benötigte Trimethylsulfonium-methylsulfat der Formel (VI) ist ebenfalls bekannt (vgl. Heterocycles 8, 397 (1977)). Es wird bei der obigen Umsetzung ebenfalls in frisch hergestelltem Zustand eingesetzt, indem man es durch Reaktion von Dimethylsulfid mit Dimethylsulfat in situ erzeugt.

Bei der Variante (α) des Verfahrens zur Herstellung der Oxirane der Formel (II) kommt als Verdünnungsmittel vorzugsweise Dimethylsulfoxid infrage.

Die Reaktionstemperaturen können bei der oben beschriebenen Verfahrensvariante (α) innerhalb eines größeren Bereiches variiert werden. Im allgemeinen arbeitet man bei Temperaturen zwischen 20°C und 80°C.

Die Durchführung des Verfahrens zur Herstellung der Oxirane der Formel (II) nach der Variante (α) und die Aufarbeitung des bei dieser Synthese anfallenden Reaktionsgemisches erfolgt nach üblichen Methoden (vgl. J.Amer.Chem.Soc. 87, 1363-1364 (1965)).

Bei der Variante (β) zur Herstellung der Oxirane der Formel (II) kommt als inertes organisches Lösungsmittel vorzugsweise Acetonitril in Betracht.

Als Base können bei der Verfahrensvariante (β) starke anorganische oder organische Basen verwendet werden. Vorzugsweise infrage kommt Natriummethylat.

Die Reaktionstemperaturen können bei der oben beschriebenen Varfahrensvariante (β) innerhalb eines bestimmten Bereiches variiert werden. Im allgemeinen arbeitet man bei Temperaturen zwischen 0°C und 60°C, vorzugsweise bei Raumtemperatur.

Die Durchführung des Verfahrens zur Herstellung der Oxirane der Formel (II) nach der Variante (β) und die Aufarbeitung des bei dieser Synthese anfallenden Reaktionsproduktes erfolgt nach üblichen Methoden (vgl. Heterocycles 8, 397 (1977)).

Die Oxirane der Formel (II) können bei dem erfindungsgemäßen Verfahren gegebenenfalls ohne Isolierung direkt weiter umgesetzt werden.

Die außerdem für das erfindungsgemäße Verfahren als Ausgangsstoffe zu verwendenden 1,2,4-Triazole sind durch die Formel (III) allgemein definiert. In dieser Formel steht M vorzugsweise für Wasserstoff, Natrium oder Kalium.

Die 1,2,4-Triazole der Formel (III) sind allgemein bekannte Verbindungen der organischen Chemie.

Als Verdünnungsmittel kommen für das erfindungsgemäße Verfahren unter den Reaktionsbedingungen inerte organische Lösungsmittel infrage. Hierzu gehören vorzugsweise Alkohole, wie z.B. Ethanol, Methoxyethanol oder Propanol; Ketone, wie z.B. 2-Butanol; Nitrile, wie z.B. Acetonitril; Ester, wie z.B. Essigester; Ether, wie z.B. Dioxan; aromatische Kohlenwasserstoffe, wie z.B. Benzol und Toluol; oder Amide, wie z.B. Dimethylformamid.

Als Basen kommen für die erfindungsgemäße Umsetzung alle üblicherweise verwendbaren anorganischen und organischen Basen in Betracht. Hierzu gehören vorzugsweise Alkalicarbonate, wie z.B. Natrium- und Kaliumcarbonat; Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise zwischen 60°C und 150°C.

Bei der Durchführung des erfindungsgemäßen Verfahrens setzt man vorzugsweise auf 1 Mol Oxiran der Formel (II) 1 bis 2 Mol 1,2,4-Triazol und gegebenenfalls katalytische bis 2-molare Mengen an Base ein. Die Isolierung der Endprodukte erfolgt in allgemein üblicher Weise.

Die nach dem erfindungsgemäßen Verfahren erhältlichen Verbindungen der Formel (I) können in Säureadditions-Salze bzw. Metallsalz-Komplexe überführt werden.

Zur Herstellung von physiologisch verträglichen Säureadditions-Salzen der Verbindungen der Formel (I) kommen vorzugsweise folgende Säuren infrage : Die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure,sowie Sulfonsäuren, wie z.B. P-Toluolsulfonsäure und 1,5-Naphthalindisulfonsäure.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise Salze von Metallen der II. bis IV. Haupt- und der I. bis II. sowie IV. bis VIII. Nebengruppe infrage, wobei Kupfer, Zink, Mangan, Magnesium, Zinn, Eisen und Nickel beispielhaft genannt seien. Als Anionen der Salze kommen solche in Betracht, die sich vorzugsweise von folgenden Säuren ableiten : Halogenwasserstoffsäuren, wie z.B. die Chlorwasserstoffsäure und die Bromwasserstoffsäure, ferner Phosphorsäure, Salpetersäure und die Schwefelsäure.

Die Metallsalz-Komplexe von Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung von Reiskrankheiten, wie Pyricularia oryzae und Pellicularia sasakii, von Getreidekrankheiten, wie Leptosphaeria nodorum, Puccinia recondita, Mehltau, Cochliobolus sativus und Pyrenophora teres, sowie von Gemüse- und Obstkrankheiten, wie Sphaerotheca fuliginea und Venturia inaequalis, eingesetzt werden.

Hervorzuheben ist, daß die erfindungsgemäßen Stoffe nicht nur eine protektive Wirkung haben, sondern teilweise auch systemisch wirksam sind. So gelingt es, Pflanzen gegen Pilzbefall zu schützen, wenn man den Wirkstoff über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und /oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkyl-naphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, starke polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff



BAD ORIGINAL

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material, wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäureester, Polyoxyethylen-Fettalkohol-ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Oele sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

0142654

Herstellungsbeispiele :

Beispiel 1 :

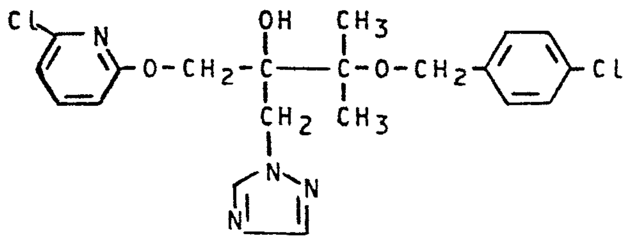

18,4 g (0,05 Mol) rohes 2-[2-(4-Chlorbenzyloxy)-prop-2-yl]-2-(6-chlorpyridin-2-yloxymethyl)-oxiran werden in 7 g (0,1 Mol) 1,2,4-Triazol und 2 g Kaliumcarbonat in 100 ml Acetonitril einige Stunden unter Rückfluß erhitzt (bis zum völligen Verbrauch des Epoxids). Anschließend wird das Reaktionsgemisch auf Wasser gegeben und mit Methylenchlorid extrahiert. Die organische Phase wird über Natriumsulfat getrocknet und eingeengt.

Man erhält 19 g (87 % der Theorie) 2-(4-Chlorbenzyloxy)-3-(6-chlorpyridin-2-yloxymethyl)-2-methyl-4-(1,2,4-triazol-1-yl)-3-butanol vom Brechungsindex $n_D^{20}$= 1,5618.

Herstellung des Ausgangsproduktes :

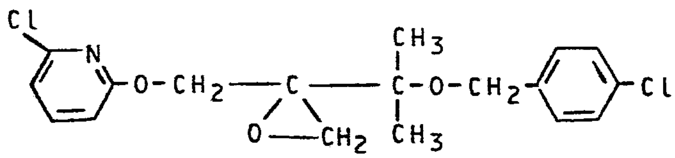

1,5 g (0,05 Mol) 80 %-iges Natriumhydrid werden bei 10°C unter Rühren portionsweise zu einer Mischung von 11 g (0,05 Mol) Trimethylsulfoxoniumjodid und 60 ml Dimethylsulfoxid gegeben. Man läßt die Reaktionsmischung 15 Minuten rühren, tropft dann 17,7 g (0,05 Mol) 3-(4-Chlorbenzyloxy)-1-(6-chlorpyridin-2-yloxy)-3-methyl-2-butanon in wenig Dimethylsulfoxid zu und erwärmt 1 Stunde auf 60°C. Unter Stickstoffatmosphäre gießt man anschließend in Wasser und extrahiert mit Methylenchlorid. Die organische Phase wird über Natriumsulfat getrocknet und eingeengt. Das so erhaltene rohe 2-[2-(4-Chlorbenzyloxy)-prop-2-yl]-2-(6-chlorpyridin-2-yloxymethyl)-oxiran wird direkt weiter umgesetzt.

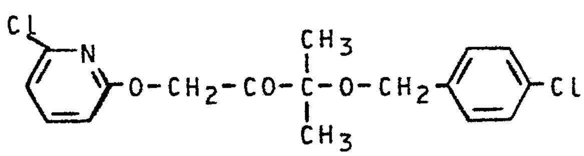

31 g (0,1 Mol) 1-Brom-3-(4-chlorbenzyloxy)-3-methyl-2-butanon in 200 ml Aceton werden mit 14 g (0,1 Mol) Kaliumcarbonat und 13 g (0,1 Mol) 6-Chlor-2-hydroxypyridin 6 Stunden unter Rückfluß erhitzt. Danach wird das Reaktionsgemisch in Wasser gegeben und mit Methylenchlorid

extrahiert. Die organische Phase wird über Natriumsulfat getrocknet und eingeengt.

Man erhält 31,5 g (89 % der Theorie) 3-(4-Chlorbenzyloxy)-1-(6-chlorpyridin-2-yloxy)-3-methyl-2-butanon als helles Oel.

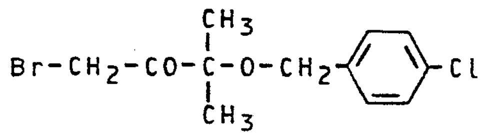

22,7 g (0,1 Mol) 3-(4-Chlorbenzyloxy)-3-methyl-2-butanon in 250 ml Methylenchlorid werden bei Raumtemperatur tropfenweise mit 16,7 g (0,1 Mol) Brom versetzt. Nach vollständiger Entfärbung gießt man die Reaktionsmischung in Wasser. Die organische Phase wird abgetrennt, mit Natriumhydrogencarbonat-Lösung neutral gewaschen und eingeengt. Der Rückstand wird destilliert.

Man erhält 18 g (60 % der Theorie) 1-Brom-3-(4-chlorbenzyloxy)-3-methyl-2-butanon vom Siedepunkt 128°C bis 138°C/ 0,03 Torr.

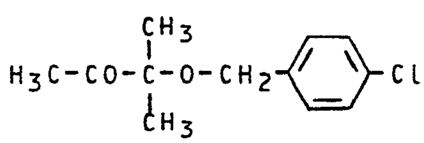

134 g (0,5 Mol) 2-(4-Chlorbenzyloxy)-3,3-dimethoxy-2-methyl-butan werden in 1 l Toluol mit 100 g Ionenaustauscher (Levatit SP C118) und 200 ml Wasser 8 Stunden bei 60°C gerührt. Nach dem Absaugen des Ionenaustauschers wird die organische Phase abgetrennt, mit Wasser gewaschen und in Vakuum eingeengt. Der Rückstand wird destilliert. Man erhält 98 g (87 % der Theorie) 3-(4-Chlorbenzyloxy)-3-methyl-2-butanon vom Siedepunkt 95°C bis 100°C/0,03 Torr.

In analoger Weise und entsprechend dem erfindungsgemäßen Verfahren werden die nachfolgenden Verbindungen der allgemeinen Formel (I) erhalten :

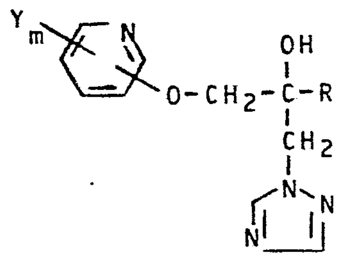

(I)

| Beisp. Nr. | 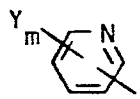 | R | Fp.(°C) bzw. $n_D^{20}$ |
|---|---|---|---|
| 2 | 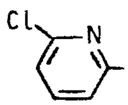 | 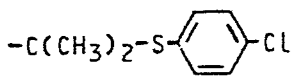 | 85 - 90 |
| 3 | 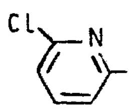 | 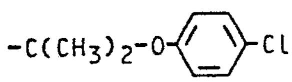 | 72 - 75 |
| 4 | 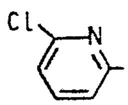 | 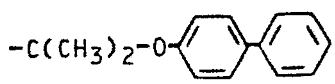 | 1,5848 |
| 5 | 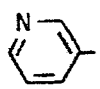 | $-C(CH_2F)_2-CH_3$ | 1,5088 |
| 6 | 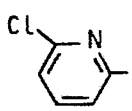 | 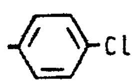 | 1,5523 |
| 7 | 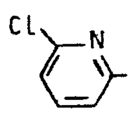 | 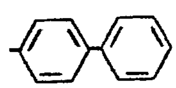 | 133 |

Verwendungsbeispiele :

In den nachfolgenden Verwendungsbeispielen werden die nachstehend angegebenen Substanzen als Vergleichsverbindungen eingesetzt :

(A) 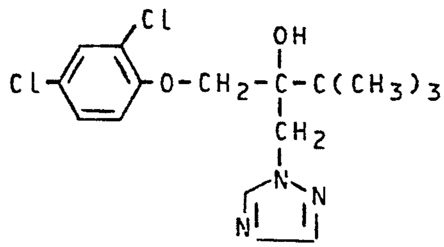

(B) 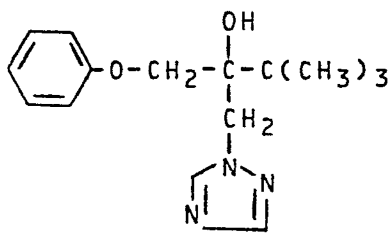

(C) 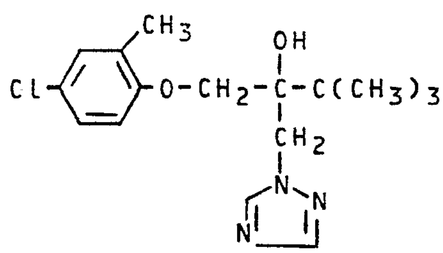

(D) 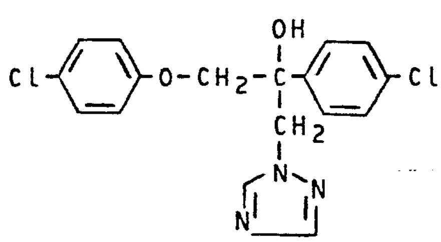

Beispiel A

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 7, 1 und 2.

0142654

Beispiel B

Pyricularia-Test (Reis) / systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 6, 7 und 3.

Beispiel C

Pellicularia-Test (Reis)

Lösungsmittel: 12,5 Gewichtsteile Aceton
Emulgator: 0,3 Gewichtsteile Alkylarlypolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf Wirksamkeit werden junge Reispflanzen im 3- bis 4-Blattstadium tropfnaß gespritzt. Die Pflanzen verbleiben bis zum Abtrocknen im Gewächshaus. Anschließend werden die Pflanzen mit Pellicularia sasakii inokuliert und bei 25°C und 100 % relativer Luftfeuchtigkeit aufgestellt.

5 bis 8 Tage nach der Inokulation erfolgt die Auswertung des Krankheitsbefalles.

Eine deutliche Überlegenheit in der Wirksamkeit gegenüber dem Stand der Technik zeigen bei diesem Test z.B. die Verbindungen gemäß folgender Herstellungsbeispiele: 6 und 7.

Beispiel D

Puccinia-Test (Weizen) / protektiv

Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit einer Sporensuspension von Puccinia recondita in einer 0,1 %igen wäßrigen Agarlösung inokuliert. Nach Antrocknen besprüht man die Pflanzen mit der Wirkstoffzubereitung taufeucht. Die Pflanzen verbleiben 24 Stunden bei 20°C und 100 % rel. Luftfeuchtigkeit in einer Inkubationskabine.

Die Pflanzen werden in einem Gewächshaus bei einer Temperatur von ca. 20°C und einer relativen Luftfeuchtigkeit von ca. 80 % aufgestellt, um die Entwicklung von Rostpusteln zu begünstigen.

10 Tage nach der Inokulation erfolgt die Auswertung.

Eine deutliche Überlegenheit in der Wirksamkeit gegenüber dem Stand der Technik zeigen bei diesem Test z. B. die Verbindungen gemäß folgender Herstellungsbeispiele: 7 und 1.

Beispiel E :

Leptosphaeria nodorum-Test (Weizen) / protektiv

Lösungsmittel: 100 Gewichtsteile Dimethylformamid
Emulgator : 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Leptosphaeria nodorum besprüht. Die Pflanzen verbleiben 48 Stunden bei 20°C und 100 % relativer Luftfeuchtigkeit in einer Inkubationskabine.
Die Pflanzen werden im Gewächshaus bei einer Temperatur von ca. 15°C und einer relativen Luftfeuchtigkeit von ca. 80% aufgestellt.

10 Tage nach der Inokulation erfolgt die Auswertung.

Eine deutliche Ueberlegenheit in der Wirksamkeit gegenüber dem Stand der Technik zeigen bei diesem Test z.B. die Verbindungen gemäß folgender Herstellungsbeispiele: 6.

Beispiel F

Sphaerotheca-Test (Gurke) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 6.

Beispiel G

Venturia-Test (Apfel) / protektiv

Lösungsmittel: 4,7 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers (Venturia inaequalis) inokuliert und verbleiben dann 1 Tag bei 20°C und 100 % relativer Luftfeuchtigkeit in einer Inkubationskabine.

Die Pflanzen werden dann im Gewächshaus bei 20°C und einer relativen Luftfeuchtigkeit von ca. 70 % aufgestellt.

12 Tage nach der Inokulation erfolgt die Auswertung.

Eine deutliche Überlegenheit in der Wirksamkeit gegenüber dem Stand der Technik zeigen in diesem Test z.B. die Verbindungen gemäß folgender Herstellungsbeispiele: 6.

Patentansprüche

1. Triazolylmethyl-pyridyloxymethyl-carbinol-Derivate der allgemeinen Formel (I),

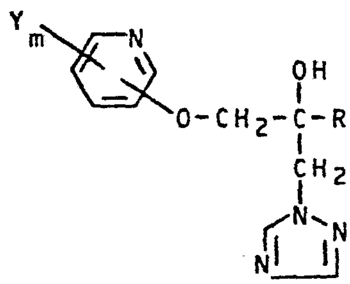

(I)

in welcher

R für gegebenenfalls substituiertes Alkyl, gegebenenfalls substituiertes Cycloalkyl oder gegebenenfalls substituiertes Phenyl steht,

Y für Halogen, Alkyl, Alkoxy oder Cyano steht und

m für die Zahlen 0, 1, 2, 3 oder 4 steht,

und deren Säureadditions-Salze und Metallsalz-Komplexe.

2. Verbindungen der allgemeinen Formel (I) in Anspruch 1, wobei
R für geradkettiges oder verzweigtes Alkyl mit 1 bis 4 Kohlenstoffatomen steht, für gegebenenfalls einfach oder mehrfach, gleich oder verschieden durch Halogen, Alkyl mit 1 bis 4 Kohlenstoffatomen und Alkoxy mit 1 bis 2 Kohlenstoffatomen substituiertes Cycloalkyl mit 3 bis 7 Kohlenstoffatomen steht, ferner für gegebenenfalls einfach oder mehrfach, gleich oder verschieden substituiertes Phenyl steht, wobei als Substituenten die unten bei $R^3$ angegebenen Phenylsubstituenten zu nennen sind, sowie für die Gruppierungen

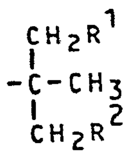 und 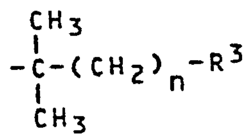 steht, worin

$R^1$ für Wasserstoff oder Halogen steht,

$R^2$ für Halogen steht und

$R^3$ für Alkyl, Alkoxy und Alkylthio mit jeweils 1 bis 4 Kohlenstoffatomen steht, für Halogenalkoxy und Halogenalkylthio mit jeweils 1 bis 2 Kohlenstoffatomen und 1 bis 5 gleichen oder verschiedenen Halogenatomen steht, ferner für Alkenyl mit 2 bis 6 Kohlenstoffatomen, für Alkoxycarbonyl mit 1 bis 4 Kohlenstoffatomen im Alkylteil, für Cyano sowie für gegebenenfalls einfach oder mehrfach substituiertes Phenyl, Phenoxy, Phenylthio, Phenylalkoxy mit 1 bis 4 Kohlenstoffatomen im Alkylteil und Phenylalkylthio mit 1 bis 4 Kohlenstoffatomen im Alkylteil steht, wobei jeweils als Phenylsubstituenten zu nennen sind: Halogen, Alkyl mit 1 bis 4 Kohlenstoffatomen, Alkoxy und Alkylthio mit jeweils 1 bis 2 Kohlenstoffatomen, Halogenalkyl, Halogenalkoxy und Halogenalkylthio mit jeweils 1 bis 2 Kohlenstoff- und 1 bis 5 gleichen oder verschiedenen Halogenatomen, Cyclohexyl, Dialkylamino mit 1 bis 4 Kohlenstoffatomen in

jedem Alkylteil, Nitro, Cyano, Alkoxycarbonyl mit 1 bis 4 Kohlenstoffatomen im Alkylteil, sowie gegebenenfalls durch Halogen substituiertes Phenyl, und

n für die Zahlen 0, 1 oder 2 steht,

Y für Halogen, geradkettiges oder verzweigtes Alkyl und/ oder Alkoxy mit jeweils 1 bis 4 Kohlenstoffatomen sowie für Cyano steht, und

m für die Zahlen 0, 1, 2, 3 oder 4 steht.

3. Verbindungen der allgemeinen Formel (I) in Anspruch 1, wobei

R für tert.-Butyl oder Isopropyl steht, für jeweils gegebenenfalls einfach bis dreifach, gleich oder verschieden durch Methyl, Ethyl, Isopropyl, Methoxy und Ethoxy substituiertes Cyclopropyl, Cyclopentyl oder Cyclohexyl steht, ferner für gegebenenfalls einfach bis dreifach, gleich oder verschieden durch Fluor, Chlor, Methyl, Trifluormethyl, Phenyl und Chlorphenyl substituiertes Phenyl steht, sowie für die Gruppierungen

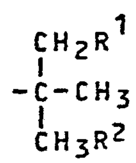 und 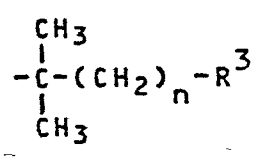 steht, wobei

$R^1$ für Wasserstoff, Fluor oder Chlor steht,

$R^2$ für Fluor oder Chlor steht,

$R^3$ für Methyl, Ethyl, Propyl, Methoxy, Ethoxy, Methylthio, Ethylthio, Trifluormethoxy, Trifluormethylthio, Vinyl, Methoxycarbonyl, Ethoxycarbonyl, Cyano sowie für jeweils gegebenenfalls durch Fluor, Chlor, Methyl, Ethyl, Methoxy, Methylthio, Trifluormethyl, Trifluormethoxy, Trifluormethylthio, Dimethylamino, Methoxycarbonyl und Ethoxycarbonyl substituiertes Phenyl, Phenoxy, Phenylthio, Phenylmethoxy oder Phenylmethylthio steht, und

n für die Zahlen 0, 1 oder 2 steht,

Y für Fluor, Chlor, Brom, Iod, Methyl, Ethyl, Methoxy oder Cyano steht, und

m für die Zahlen 0, 1, 2, 3 oder 4 steht.

4. Verbindungen der allgemeinen Formel (I) in Anspruch 1, wobei

R für tert.-Butyl oder für gegebenenfalls einfach bis dreifach, gleich oder verschieden durch Fluor, Chlor, Methyl, Trifluormethyl, Phenyl und Chlorphenyl substituiertes Phenyl steht, sowie auch für die Gruppierungen

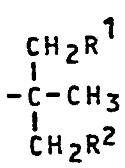 und 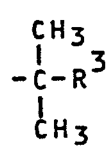 steht, wobei

$R^1$ für Wasserstoff, Fluor oder Chlor steht,

$R^2$ für Fluor oder Chlor steht und

$R^3$ für jeweils gegebenenfalls einfach bis zweifach, gleich oder verschieden durch Fluor, Chlor, Methyl, Methoxy, Methylthio, Trifluormethyl, Trifluormethoxy, Trifluormethylthio, Methoxycarbonyl und Ethoxycarbonyl substituiertes Phenoxy, Phenylthio, Phenylmethoxy oder Phenylmethylthio steht,

Y für Fluor, Chlor, Brom oder Iod steht, und

m für die Zahlen 0, 1, 2 oder 3 steht.

5. Verfahren zur Herstellung von Triazolylmethyl-pyridyloxymethyl-carbinol-Derivaten der allgemeinen Formel (I),

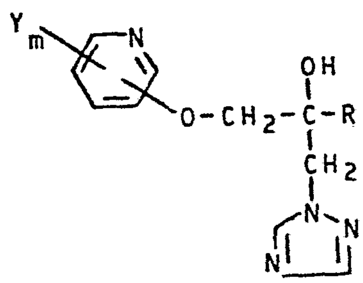

(I)

in welcher

R für gegebenenfalls substituiertes Alkyl, gegebenenfalls substituiertes Cycloalkyl oder gegebenenfalls substituiertes Phenyl steht,

Y für Halogen, Alkyl, Alkoxy oder Cyano steht, und

m für die Zahlen 0, 1, 2, 3 oder 4 steht,

dadurch gekennzeichnet, daß man Oxirane der Formel (II)

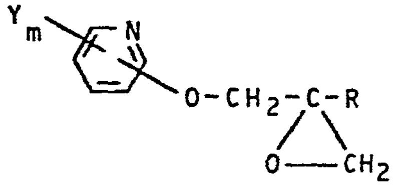

(II)

in welcher
R, Y und m die oben angegebene Bedeutung haben,

mit 1,2,4-Triazol der Formel (III),

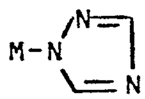

(III)

in welcher
M für Wasserstoff oder ein Alkalimetall steht,

in Gegenwart eines Verdünnungsmittels und gegebenenfalls in Gegenwart einer Base umsetzt und gegebenenfalls an die erhaltenen Verbindungen der Formel (I) anschließend eine Säure oder ein Metallsalz addiert.

6. Fungizide Mittel, gekennzeichnet durch einen Gehalt an mindestens einem Triazolylmethyl-pyridyloxymethyl-carbinol-Derivat der Formel (I) in Ansprüchen 1 und 5.

7. Verfahren zur Bekämpfung von Pilzen, dadurch gekennzeichnet, daß man Triazolylmethyl-pyridyloxymethyl-carbinol-Derivate der Formel (I) in Ansprüchen 1 und 5 auf Pilze oder ihren Lebensraum einwirken läßt.

8. Verwendung von Triazolylmethyl-pyridyloxymethyl-carbinol-Derivaten der Formel (I) in Ansprüchen 1 und 5 als Pflanzenschutzmittel.

9. Verwendung von Triazolylmethyl-pyridyloxymethyl-carbinol-Derivaten der Formel (I) in Ansprüchen 1 und 5 zur Bekämpfung von Pilzen.

10. Verfahren zur Herstellung von fungiziden Mitteln, dadurch gekennzeichnet, daß man Triazolylmethyl-pyridyloxymethyl-carbinol-Derivate der Formel (I) in Ansprüchen 1 und 5 mit Streckmitteln und/oder oberflächenaktiven Mitteln vermischt.

Europäisches Patentamt

# EUROPÄISCHER RECHERCHENBERICHT

0142654

Nummer der Anmeldung

EP 84110734.5

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | EP - A1 - 0 061 835 (I.C.I.)<br>* Formel I; Anspruch 18, Verbindung 112 *<br>-- | 1,6 | C 07 D 401/12<br>A 01 N 43/64 |
| A | EP - A1 - 0 045 016 (BAYER)<br>* Zusammenfassung *<br>-- | 1,6 | |
| A | DE - A1 - 3 000 244 (BAYER)<br>* Anspruch 1,3 *<br>-- | 1,6 | |
| A | DE - A1 - 2 908 378 (HOECHST)<br>* Anspruch 1,3 *<br>-- | 1,6 | |
| A | DE - A1 - 2 921 168 (BASF)<br>* Formel I *<br>-- | 1 | RECHERCHIERTE SACHGEBIETE (Int. Cl.4) |
| A | EP - A3 - 0 047 594 (I.C.I.)<br>* Zusammenfassung *<br>---- | 1 | C 07 D 401/00 |

Der vorliegende Recherchenbericht wurde für alle Patentansprüche erstellt.

| Recherchenort | Abschlußdatum der Recherche | Prüfer |
|---|---|---|
| WIEN | 29-11-1984 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82